# EUROPEAN PATENT APPLICATION

(11) **EP 4 628 890 A1**
(43) Date of publication of application: **08.10.2025**
(21) Application number: 23897711.0
(22) Date of filing: 27.11.2023
(51) Int. Cl.: G01N 33/48, G01N 1/10, G01N 1/38, G01N 33/68

(54) **SOLUTION AND METHOD FOR TRANSPORTING OR HOLDING BLOOD IN VERY SMALL AMOUNT**

(30) Priority: 30.11.2022 JP 2022191522
(71) Applicant: Provigate Inc., Tokyo 113-0033 (JP)
(72) Inventor: NISHI, Mitsumi, Tokyo 113-0033 (JP); MIYAUCHI, Noriko, Tokyo 113-0033 (JP); IRIE, Tomoko, Tokyo 113-0033 (JP)
(74) Representative: Michalski Hüttermann & Partner Patentanwälte mbB
(86) International application number: PCT/JP2023/042303
(87) International publication number: WO 2024/117063

(57) **Abstract**

According to an embodiment of the present disclosure, a solution for transporting or storing a blood sample is provided. The blood sample may be a microvolume blood sample. In some embodiments, the solution may contain a buffering agent and a salt as active ingredients. The solution may be configured to dilute the blood at a dilution ratio of 10 or greater.

## Description

### TECHNICAL FIELD

The present disclosure relates to a solution and a method for transporting or storing microvolume blood.

### BACKGROUND ART

In blood tests, various biomarkers present in blood are measured in various ways. Examples thereof include glycated albumin in blood. Glycated albumin is a glycation product of albumin. Albumin is a protein that is present in abundance in the body and binds to glucose at a high ratio. A degree of glycation (GA value or GA%) of glycated albumin (GA) is considered to represent an average of blood glucose levels for approximately the most recent two weeks. Accordingly, the GA value has attracted attention in recent years as an index that can be used for the control and management of blood glucose.

Desirably, the measurement of the GA value is performed more frequently than typical blood tests. Accordingly, one effective approach is to perform transportation-and-testing rather than testing at a hospital. Specifically, the user himself or herself collects microvolume blood, which may be, for example, fingertip blood, and sends it to a test facility by using a transportation method, such as postal mail. Test facilities can have a measurement instrument that can perform the test with high efficiency and high accuracy, and thus, the test facilities can provide test results to users and associated organizations.

Glucose or a different component in whole blood reacts with albumin during transportation or storage. Consequently, the final actual GA value may be affected, and, further, the measurement of the GA value may be affected.

### SUMMARY OF INVENTION

Some embodiments of the present disclosure provide a solution for transporting or storing a blood sample. In some embodiments, the blood sample may be a microvolume blood sample. In some embodiments, the solution may contain a buffering agent and a salt as active ingredients. The solution may be configured to dilute the blood at a dilution ratio of greater than 8.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the present disclosure. Accordingly, the drawings and description are to be regarded as illustrative in nature, and not as restrictive.

### BRIEF DESCRIPTION OF DRAWINGS

[Fig. 1] Fig. 1 is a graph illustrating temporal changes in the GA value, associated with different dilution ratios, that were observed in an Example.
[Fig. 2] Fig. 2 is a graph illustrating temporal changes in the GA value that were observed in two Examples.
[Fig. 3] Fig. 3 is a conceptual diagram illustrating a process of using a mail-in kit containing a diluent solution, according to an embodiment.
[Fig. 4] Fig. 4 is a conceptual diagram illustrating a process of using a mail-in kit containing a diluent solution, according to an embodiment.

### <Microvolume Blood>

The blood sample can be provided in various ways. In some embodiments, the blood sample may be capillary blood. In some embodiments, the blood sample may be collected from a vein or artery of a living body. In some embodiments, the blood sample may be collected by a puncture technique. The blood sample may be collected from a droplet (capillary blood) formed on the skin by puncturing a fingertip, an earlobe, a sole of a foot, an arm, an abdominal region, or the like. In some embodiments, the blood sample may be collected by the subject himself or herself. In some embodiments, the blood sample may be collected by a doctor, a nurse, or a medical technologist. In some embodiments, the blood sample may be collected in the vicinity of a test instrument (e.g., in a clinic where a test instrument is installed).

In some embodiments, the blood sample collected from the subject may be a microvolume sample. The microvolume blood may have a volume of a droplet (capillary blood) of the microvolume blood, where the droplet is generated on the skin by a puncture technique.

Typically, the volume of fingertip blood is between 0.5 µL and 20 µL. In some embodiments, the volume of fingertip blood is 10 µL. A predetermined volume of the blood may be collected.

The volume may be equal to or greater than a value such as 0.1 µL, 0.2 µL, 0.3 µL, 0.4 µL, 0.5 µL, 0.6 µL, 0.7 µL, 0.8 µL, 0.9 µL, 1.0 µL, 1.1 µL, 1.2 µL, 1.3 µL, 1.4 µL, 1.5 µL, 1.6 µL, 1.7 µL, 1.8 µL, 1.9 µL, or 2.0 µL.

The volume may be equal to or less than a value such as 1.0 µL, 1.5 µL, 2 µL, 3 µL, 4 µL, 5 µL, 6 µL, 7 µL, 8 µL, 9 µL, 10 µL, or 15 µL.

In some embodiments, the blood sample to be mixed with a diluent solution may be whole blood. In some embodiments, the blood sample to be mixed with the diluent solution may be a plasma or a serum.

### <Dilution Ratio>

As used in this specification, the expression "dilution ratio" or "N ×" refers to (V0 + V1)/V0 where V0 is the volume of collected blood, and V1 is the volume of the diluent solution used. For example, in an instance where 9 µL of the diluent solution is mixed with 1 µL of collected blood to provide 10 µL of a transportation solution or a storage solution, the dilution ratio is "10" or "10 ×".

The inventors discovered that during transportation or storage, albumin may react with glucose present in blood and may, consequently, be glycated, which can result in changes in the measured value. In general, it can be assumed that the rate of glycation increases in response to the frequency of physical contact between albumin and glucose. The dilution of the blood sample reduces the frequency, thereby contributing to regulating the rate of glycation. Sufficient dilution makes it possible to obtain the GA value with required accuracy over a range of a realistic transport time (e.g., several hours, 2 days, several days, or 1 week) and a realistic temperature (e.g., room temperature or ambient temperature). In some embodiments, such a sufficient dilution ratio may be employed. If the dilution ratio is excessively low, that is, if a sufficient dilution ratio is not employed, the GA value changes during transportation, and, consequently, the GA value of the subject cannot be determined with high accuracy.

The dilution ratio may be equal to or greater than a value such as 5 ×, 6 ×, 7 ×, 8 ×, 9 ×, or 10 ×. In some embodiments, the dilution ratio may be 5 × or greater. In some embodiments, the dilution ratio may be greater than 8 ×. In some embodiments, the dilution ratio may be 10 × or greater.

If the dilution ratio is excessively high, that is, if a concentration of albumin is excessively low, the measurement cannot be performed. At least a measurement method and a measurement instrument have a limit of the concentration. It is necessary that the concentration of albumin present in the mixed solution be greater than the limit of the concentration. The amount of blood collected of some subjects may be small. Principally or practically, the concentration needs to be at least within a range in which the measurement is possible.

The dilution ratio may be equal to or less than a value such as 10 ×, 15 ×, 20 ×, 25 ×, 30 ×, 40 ×, 50 ×, 60 ×, 70 ×, 80 ×, 90 ×, or 100 ×. In some embodiments, the dilution ratio may be 100 × or less. In some embodiments, the dilution ratio may be 90 × or less. In some embodiments, the dilution ratio may be 80 × or less. In some embodiments, the dilution ratio may be 50 × or less. In some embodiments, the dilution ratio may be 20 × or less.

In some embodiments, the dilution ratio may be between 5 × and 100 ×. In some embodiments, the dilution ratio may be between 8 × and 100 ×. In some embodiments, the dilution ratio may be between 9 × and 100 ×. In some embodiments, the dilution ratio may be between 10 × and 100 ×.

### <Buffering Agent>

In some embodiments, the solution may contain a buffering agent. In some embodiments, the buffering agent may be selected from the group consisting of HEPES (4-(2-hydroxyethyl)-1-piperazineethanesulfonic acid), sodium acetate, and PBS (phosphate buffered saline).

### <Salt>

In some embodiments, the solution may contain a salt. For example and without limitation, the salt may be NaCl, KCl, or the like. In some embodiments, the salt may be NaCl.

In some embodiments, the solution may be adjusted to form an isotonic solution with respect to a blood cell component. In this case, hemolysis can be inhibited. In general, hemolysis does not affect HPLC measurements. However, if components resulting from hemolysis, such as hemoglobin, are denatured or modified by heat or a lapse of time, they may affect HPLC measurements. In other cases, hemolysis affects optical measurements, such as an absorbance measurement. Accordingly, inhibiting hemolysis during transportation or storage reduces the risk of making the measurement impossible or inaccurate.

### <GA Measurement>

The present disclosure is not intended to limit the method for GA measurement. In some embodiments, the GA value may be measured by using HPLC. In some embodiments, the GA value may be measured by using an enzymatic method or the like.

In general, absorbance spectrometers that use an enzymatic method are inexpensive and do not need a large occupancy space. In addition, they allow the use of commercially available reagents and do not require, for example, condition setting or preparation of reagents. In addition, a pretreatment operation process is simple. It is sufficient to merely place a centrifuged blood collection tube as it is. However, these are based on an optical measurement, such as an absorbance measurement, and, therefore, in instances where a substance containing a coloring material, such as hemoglobin, is present, for example, in instances where whole blood is measured, correct measured values cannot be obtained. In other words, in the instance of hemolysis, an accurate measurement cannot be made.

HPLC instruments, in general, can perform a relatively accurate measurement even in the instance of hemolysis. They are dedicated instruments, and, therefore, they involve high per-unit cost, need a large occupancy space (footprint), and involve a long measurement time. However, multiple HPLC instruments may be installed in a facility so that operations can be consistently performed under appropriate control. Consequently, high accuracy can be achieved, and in addition, high efficiency and low cost can be achieved as a whole.

### <Other Ingredients>

In some embodiments, the solution may contain a buffering agent and a salt as active ingredients. In some embodiments, the solution may contain an active ingredient consisting of a buffering agent and a salt serving as active ingredients. In some embodiments, the solution may contain a buffering agent, a salt, and water. In some embodiments, the solution may consist essentially of a buffering agent, a salt, and water.

In some embodiments, the solution may contain one or more ingredients other than a buffering agent or a salt. In some embodiments, it is preferable that the solution be free of albumin and glycated albumin or of an ingredient that substantially affects the measurement. For example, it is preferable not to use certain types of preservatives. For example, it is preferable not to use ProClin (registered trademark). It is preferable not to use CMIT and/or MIT. It is preferable not to use meropenem trihydrate. Although not illustrated, the inventors discovered that these ingredients tend to provide a GA value that is higher than the actual GA value.

### <Examples>

Examples will be described below. A blood sample was diluted with a diluent solution of an embodiment of the present disclosure, and, after 0 to 2 days, the GA value was measured by using HPLC.

### <Preparation of Measurement Sample>

A fixed amount of the blood sample (10 µL, see below) was introduced into 1.5-mL tubes, into which a diluent solution (HEPES buffer) was dispensed such that desired dilution ratios could be achieved. 2 × to 120 × dilution series were prepared. The dilution ratios and the volumes of the diluent solution that was dispensed into the tubes were as follows. 2 ×: 10 µL, 5 ×: 40 µL, 8 ×: 70 µL, 10 ×: 90 µL, and 40 ×: 190 µL. In this specification, a "10-fold" or "10 × diluted solution", for example, refers to a sample prepared by mixing 10 µL of a whole blood sample with 90 µL of a diluent solution.

Venous blood was collected into a vacuum blood collection tube containing an anticoagulant. Glucose was added to the venous blood such that an amount of glucose of 1000 mg/dL could be achieved. 10 µL aliquots of the whole blood, which was slowly mixed by inversion, were dispensed into the prepared tubes, and then, the contents were mixed in a manner that could prevent hemolysis.

The samples were measured on the same day ("day 0") and measured after they were stored for 1 day ("day 1") or 2 days ("day 2") in a constant-temperature chamber at 37°C.

Prior to the measurement, blood cells were removed by centrifugation; for this, the 2 × to 8 × diluted solutions were diluted to have the same concentration as the 10 × diluted solution, by adding a diluent solution to them. For the 10 ×, 40 ×, and 120 × diluted solutions, the removal of blood cells by centrifugation was performed without adding any diluent solution to them. Next, half of an amount of an eluent was added to the 2 × to 10 × diluted solutions, and a 20 µL aliquot of each of the solutions was injected into an HPLC instrument. No diluent solution was added to the 40 × and 120 × diluted solutions, and ethanol was added to them to achieve a final concentration of 4%; thus, 40 µL and 100 µL aliquots of the respective solutions were injected into the HPLC instrument.

### <HPLC Measurement>

The measurement conditions were selected by referring to "GA Detection by Using HPLC Method", J Chromatogr 597, 271-275 (1992). The columns used were AEX: Shodex-Asahipak ES-502N (7.5 mm ID × 100 mm) (Showa Denko K.K.) and BAC: TSKgel Boronate-5PW (4.6 mm ID × 100 mm) (Tosoh Corporation). The eluents used were glycine (JIS special grade), magnesium chloride hexahydrate (JIS special grade), D(-)-sorbitol (Wako 1st Grade), ethanol (99.5%, JIS special grade), tris(hydroxymethyl)aminomethane (Tris, biotechnology grade), and EDTA-2Na. The analysis was performed with a Prominence HPLC system (Shimadzu Corporation) provided with a system controller.

### <Example 1>

The diluent solution prepared was as follows:

| | |
|---|---|
| HEPES | 10 mM |
| NaCl | 150 mM |
| Volume | 90 µL |
| pH | 8.0 |

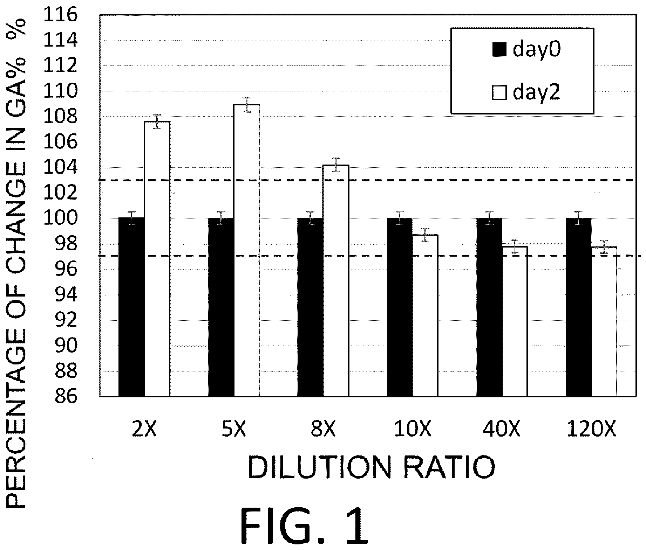

Fig. 1 shows relative GA values obtained on "day 0", which is the same day as the day of preparation, and on "day 2", which is two days later. A ratio (percentage of change in the GA%) of the GA% of "day 2" to the GA% of "day 0", which was taken as 100%, was determined for each of the dilution ratios.

In the cases of 2 × to 8 ×, the GA% significantly changed with time. One possible reason for this is that glucose present in the samples reacted with albumin, which resulted in an increase in the degree of glycation of albumin. Even in the case of 8 × dilution, an increase in the GA% with time was observed; however, the rate of increase was suppressed compared to the cases of 5 × or higher concentrations. It was also found that the degree of dilution of 8 × has an effect of inhibiting glycation. On the other hand, in the cases of 10 × or thinner diluted solutions, the change in the GA% with time was small. This result suggests that with these dilution ratios, the increase in the degree of glycation of albumin due to a reaction between glucose and albumin was suppressed or did not cause substantial problems.

In Fig. 1, a threshold was set to be ±3% as an example. Specifically, in instances where the percentage of change in the GA% was 97% to 103% (100±3%), it was determined that the GA% substantially did not change. This threshold is an example and may be a different value. The threshold may be determined based on a measurement accuracy, a desired accuracy, or the like. In the cases of 10 × to 120 ×, the extent of change was less than 3%, that is, no greater than approximately 2%.

If the dilution ratio is high, the HPLC measurement may not be properly carried out. However, in an experiment performed by the inventors, even a dilution ratio of 125 × did not cause problems.

In some embodiments, the upper limit of the dilution ratio may be determined by the amount of the diluent solution that is actually used. A kit for transporting a blood sample may be, for example, a container (e.g., a tube) for storing the mixed solution of the diluent solution and the blood sample. In an example, the volume of the fingertip blood to be collected is approximately 10 µL, and the volume of the tube is approximately 1.5 mL. In this instance, the volume of the diluent solution that can be introduced into the tube is approximately 1.4 mL at most, and thus, the dilution ratio is approximately 140 ×. The volume of the diluent solution that is introduced into the tube may be smaller than this, if, for example, the transfer of the blood from a blood collection kit into the tube is taken into account. The maximum dilution ratio mentioned above is an example and may be determined based on other transport or storage conditions, including a weight and a size.

### <Example 2>

In this Example, the diluent solution prepared was as follows.

| | |
|---|---|
| Sodium acetate | 100 mM |
| NaCl | 80 mM |
| Volume | 90 µL |
| pH | 8.4 |

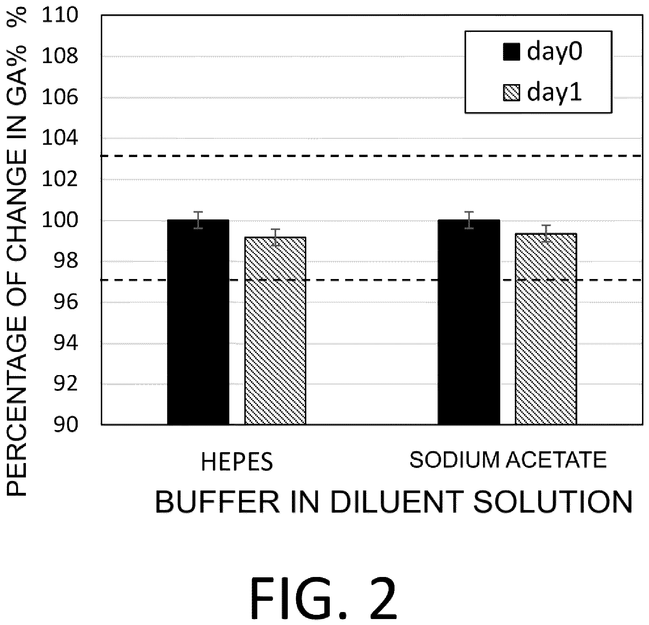

A whole blood sample was diluted 10 fold with the diluent solution of this Example, which contained sodium acetate as a buffer, and then, the percentage of change in the GA value was measured. Fig. 2 shows the results in comparison with those of the diluent solution of Example 1, which contained HEPES.

As can be seen from Fig. 2, even in the instance where sodium acetate was used as the buffer, the temporal change in the GA value was regulated to be within an acceptable range, as in the instance in which HEPES was used as the buffer.

### <Kit for Storing and Transporting Collected Blood and Diluent Solution>

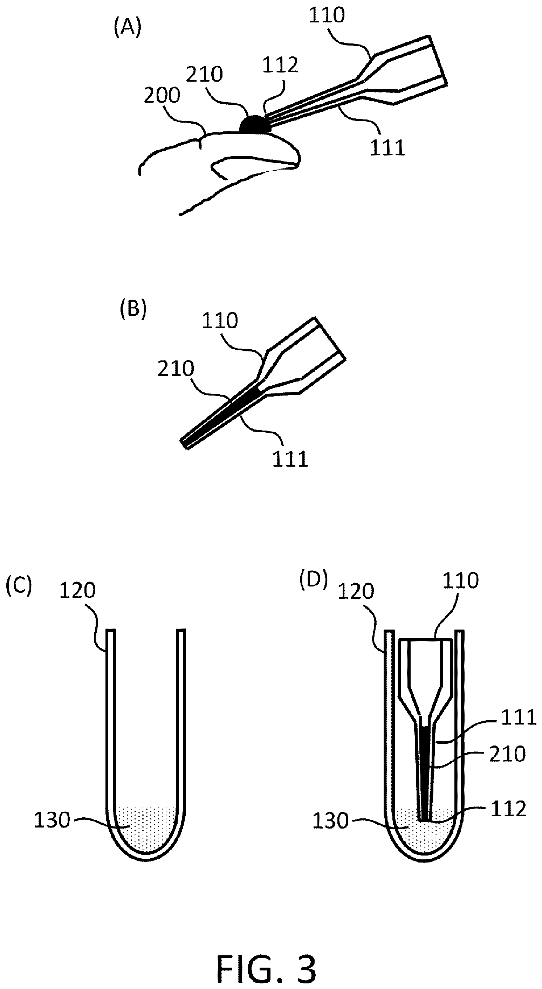

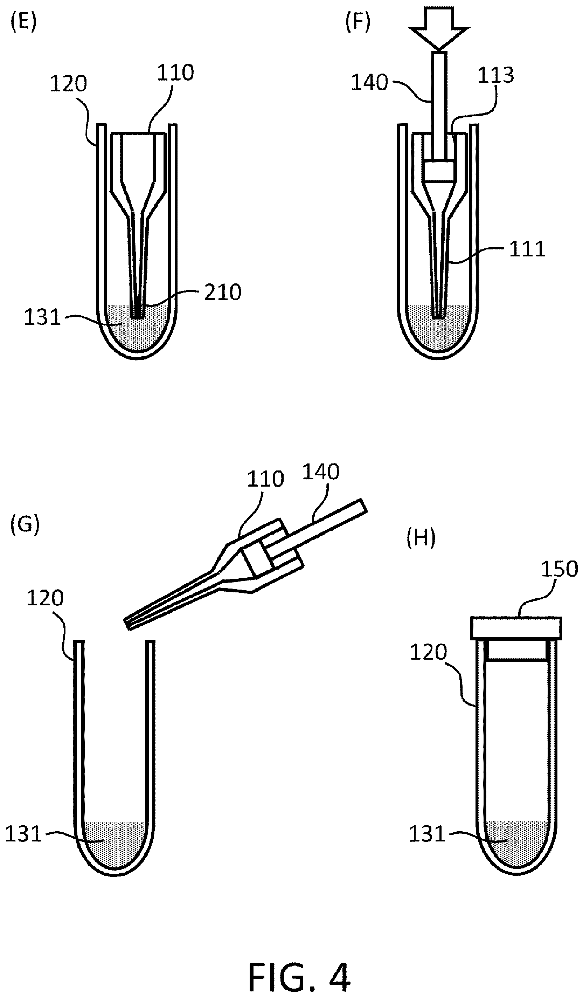

A method for storing or transporting fingertip blood, according to an embodiment, will be described with reference to Figs. 3 and 4. Blood (fingertip blood) 210 is drawn from a body surface 200 of a test subject (subject or user) by using a puncture device (not illustrated), such as a lancet. A blood collection tube 110 has a tubular structure with a portion thereof being a capillary tube 111. An end opening 112 of the capillary tube 111 is brought into contact with the fingertip blood 210 (A). The fingertip blood 210 is drawn into the capillary tube 110 by capillary action (B).

A tube 120, which contains a diluent solution 130, is prepared (C). The blood collection tube 110 containing the collected blood 210 is inserted into the tube 120 with the capillary tube being positioned downward (D). In this process, it is preferable that the end 112 of the capillary tube 111 reach a surface of the diluent solution 130 within the tube 120. Accordingly, the blood 210 within the capillary tube 111 drops into the diluent solution. It is necessary to wait for most of the blood in the capillary tube to drop into the diluent solution. As a result of the transfer of the blood 210 into the diluent solution 130, a mixed solution 131 is formed (E).

The blood collection tube 110 has a tubular structure, in which the capillary tube 111, formed at one end, is coaxial with and fluidly connected to a cylinder 113, formed at another end.

A plunger 140 is inserted into the cylinder 113 of the blood collection tube 110 from above. This increases pressure within the cylinder 113, thereby forcing the liquid out of the capillary tube 111 toward the outside. Accordingly, the remainder of the blood 210 is forcibly forced toward the outside (F). Accordingly, the amount of the remainder of the blood sample 210 within the capillary tube 111 can be reduced, which enables effective use of the blood sample for the measurement.

After the remaining blood sample 210 has been sufficiently forced out of the blood collection tube 110, the plunger 140 is pulled to remove the blood collection tube 110, which is coupled to the plunger 140, from the tube 120 (G). As a result, the mixed solution 131 of the blood sample and the diluent solution is left within the tube 120. If the blood collection tube remains in the tube, the blood sample may stick to the blood collection tube when rocking occurs during a transport or storage procedure. The removal of residues from the blood collection tube and/or the removal of the blood collection tube itself from the tube can, for example, increase efficiency for recovering the measurement target present in the blood sample and in addition, for example, lowers the risk of hemolysis.

The tube 120 is closed with a cap 150. The mixed solution 131 of the blood sample and the diluent solution is sealed in the tube 120. In this state, the mixed solution 131 is transported and/or stored in the tube 120.

Typical blood test kits (e.g., Demecal (registered trademark), Leisure, Inc.) have factors that hinder HPLC measurements. Such solutions are formulated such that blood components can be separated so that the problem of hemolysis can be avoided. Because of the separation or a large number of measurements that are to be performed, a large amount of a blood sample, for example, in an amount of 50 µL, is necessary. Furthermore, if a plasma separation filter is used, a portion of the blood remains on the filter. Consequently, a sufficient amount of the separated liquid cannot be obtained from the microvolume blood.

In some embodiments, the microvolume blood that is transported or stored may be whole blood. After the blood is collected, the microvolume whole blood may be transported or stored. Plasma separation need not be performed. In many methods for transporting and storing a blood sample for the analysis of a substance other than the blood cell component, it has been common to perform plasma separation in advance. In the present disclosure, contrary to those methods, a technique is provided for transporting or storing microvolume blood without performing plasma separation. In some embodiments, the collected microvolume blood may be diluted. Dilution facilitates the handling of the microvolume blood.

Some embodiments of the present disclosure do not require the separation of blood components. Some embodiments of the present disclosure do not require plasma separation filters. In this case, it is possible to avoid loss in the amount of collection of blood due to the blood remaining on a plasma separation filter. Components, such as a syringe, for passing the collected blood through the filter are not necessary. It is also possible to avoid loss in the amount of collection due to blood sticking to the components and a surface of the interior of the syringe. Accordingly, the collected microvolume blood can be efficiently used.

In the present disclosure, a measurement method that is not affected by hemolysis may be used, for example. HPLC, for example, may be used. A GA measurement that uses HPLC, for example, is unlikely to be affected by hemolysis. Measurements of the GA value with a small amount of blood (microvolume blood) after transportation are made possible. The GA value is not an absolute value, as opposed to, for example, concentrations, but is a relative value, which is a ratio of an amount of glycated albumin to the total amount of albumin. Accordingly, when the amount of collection is small, such an amount may have an influence on an accuracy (e.g., deviation) of concentration measurements but has a relatively small influence on the GA value measurement.

The GA value typically changes at intervals of approximately 1 to 2 weeks and, therefore, can be used to control blood glucose by periodically measuring the GA value at intervals of 1 to 2 weeks. Accordingly, less invasive or minimally invasive blood collection is effective. Collecting a small amount of blood reduces invasiveness to the user, which is a physical or psychological burden. For example, a lancet with a thin needle can be used to reduce pain. Furthermore, for example, work burdens can be avoided or reduced, and examples of the work burdens include operations of performing puncturing to squeeze out blood; repeated collection; operations of separating blood components; and retention of a state in which the plasma is separated. In addition, complex part structures are not necessary, which can reduce the cost of the kit and the cost of measurement per run.

Note that in some embodiments of the present disclosure, blood components may be separated from one another. The microvolume blood that is transported or stored may be a plasma or serum component. In some embodiments of the present disclosure, the solution may contain an ingredient that avoids or reduces hemolysis. The collected microvolume blood tends to dry, coagulate, and/or hemolyze unless it is diluted. These phenomena can significantly affect the measurement. Accordingly, there is a risk that the number of blood samples that are not suitable for the measurement may increase. In some embodiments of the present disclosure, it is possible to reduce or avoid such risks by performing appropriate dilution.

According to an embodiment of the present disclosure, the solution is free of albumin and glycated albumin or of an ingredient that substantially affects the measurement.
For example, the solution does not contain certain types of preservatives. For example, the solution does not contain ProClin (registered trademark), CMIT, MIT, and/or meropenem trihydrate.

The present disclosure includes the following embodiments.

### A001

A solution for transporting or storing microvolume blood, the solution comprising a buffering agent and a salt as active ingredients,
the solution being configured to dilute the microvolume blood at a dilution ratio of greater than 8.

### A001b

A solution for transporting or storing microvolume blood, the solution comprising a buffering agent and a salt as active ingredients,
the solution being configured to dilute the microvolume blood at a dilution ratio of substantially 10 or greater.

### A011

The solution according to A001 or any embodiment, wherein the microvolume blood is capillary blood.

### A012

The solution according to A001, A011, or any embodiment, wherein the microvolume blood is collected by a puncture technique.

### A013

The solution according to any one of A001 to A012 or any embodiment, wherein the microvolume blood is collected from a fingertip, an earlobe, a sole of a foot, an arm, or an abdominal region.

### A014

The solution according to any one of A001 to A013 or any embodiment, wherein the microvolume blood is in an amount of 0.5 to 20 µL.

### A015

The solution according to any one of A001 to A014 or any embodiment, wherein the microvolume blood is whole blood.

### A021

The solution according to any one of A001 to A014 or any embodiment, wherein the dilution ratio is 9 or greater.

### A022

The solution according to any one of A001 to A021 or any embodiment, wherein the dilution ratio is 10 or greater.

### A031

The solution according to any one of A001 to A022 or any embodiment, wherein the buffering agent is selected from the group consisting of HEPES, sodium acetate, and PBS. A041

The solution according to any one of A001 to A031 or any embodiment, wherein the salt is selected from the group consisting of NaCl, KCl, MgCl, and CaCl.

### A051

The solution according to any one of A001 to A041 or any embodiment, wherein the active ingredients are adjusted such that the solution forms an isotonic solution with respect to a blood cell component.

### A061

The solution according to any one of A001 to A051 or any embodiment, wherein the solution is used to measure a GA value, the GA value being measured after transportation or after a period of storage.

### B001

A method comprising:
providing a blood collection tube comprising a capillary tube and a cylinder fluidly connected to the capillary tube;
providing a tube holding a diluent solution;
collecting fingertip blood into the blood collection tube comprising the capillary tube;
introducing the blood collection tube, which holds the fingertip blood, into the tube in a manner that allows an end of the capillary tube to come into contact with the diluent solution;
waiting for the fingertip blood present in the blood collection tube to drop into the diluent solution;
inserting a plunger into the cylinder to force out a liquid present in the capillary tube;
removing the plunger and the blood collection tube from the tube; and
closing the tube to seal a mixed solution of the diluent solution and the fingertip blood in the tube.

### C001

A kit for sending microvolume blood by postal mail, the kit comprising the solution according to any one of A001 to A061 or any embodiment.

### C011

A kit comprising:
a) the solution according to any one of A001 to A061 or any embodiment; and
b) a tube configured to contain the solution and receive microvolume blood.

### C012

The kit according to C011 or any embodiment, further comprising c) a blood collection tube comprising a capillary tube and configured to collect a predetermined volume of blood.

### C013

The kit according to C011, C012, or any embodiment, wherein an amount of the solution is 90 µL, and the volume of blood collectable by the blood collection tube is 10 µL.

### C014

The kit according to any one of C001 to C013 or any embodiment, further comprising d) a plunger configured to be inserted into the blood collection tube to force blood out of the capillary tube.

### C015

The kit according to any one of C001 to C013 or any embodiment, further comprising e) a blood collection puncture device.

### C016

The kit according to any one of C001 to C015 or any embodiment, wherein an amount of the solution is 90 µL, and the microvolume blood is blood obtained by puncturing.

### D001

A method for transporting or storing microvolume blood, the method comprising:
providing the solution according to any one of A001 to A061 or any embodiment;
collecting microvolume blood; and
preparing a diluent solution by mixing the microvolume blood with the solution.

### D002

The method according to D001 or any embodiment, further comprising transporting the prepared diluent solution from a location at which the microvolume blood was collected to a different location.

While several embodiments and examples of the present disclosure have been described above, these embodiments and examples illustratively describe the present disclosure. For example, the above embodiments are those described in detail so that the present disclosure can be clearly described, and, if necessary, additional modifications may be made to dimensions, configurations, materials, and circuits. Note that the scope of the present disclosure encompasses embodiments in which one or more of the above-described features of the present disclosure are desirably combined. It is intended that the appended claims cover numerous modifications to the embodiments within the technical spirit of the present disclosure. Accordingly, it is to be understood that the embodiments and examples disclosed herein have been presented by way of illustration and should not be considered as limiting the scope of the present disclosure.

## Claims

1. A solution for transporting or storing microvolume blood, the solution comprising a buffering agent and a salt as active ingredients,
the solution being configured to dilute the microvolume blood at a dilution ratio of greater than 8.

2. The solution according to Claim 1, wherein the microvolume blood is capillary blood.

3. The solution according to Claim 1, wherein the microvolume blood is whole blood.

4. The solution according to Claim 1, wherein the microvolume blood is in an amount of 0.5 to 20 µL.

5. The solution according to Claim 1, wherein the dilution ratio is substantially 10 or greater.

6. The solution according to Claim 1, wherein the buffering agent is selected from the group consisting of HEPES, sodium acetate, and PBS.

7. The solution according to Claim 1, wherein the salt is selected from the group consisting of NaCl, KCl, MgCl, and CaCl.

8. The solution according to Claim 1, wherein the active ingredients are adjusted such that the solution forms an isotonic solution with respect to a blood cell component.

9. The solution according to Claim 1, wherein the solution is used to measure a GA value, the GA value being measured after transportation or after a period of storage.

10. A kit for sending microvolume blood by postal mail, the kit comprising the solution according to any one of Claims 1 to 9.

11. A kit comprising:
a) the solution according to any one of Claims 1 to 9; and
b) a tube configured to contain the solution and receive microvolume blood.

12. The kit according to Claim 11, further comprising c) a blood collection tube configured to collect a predetermined volume of blood.

13. The kit according to Claim 11 or 12, wherein an amount of the solution is 90 µL, and the volume of blood collectable by the blood collection tube is 10 µL.

14. The kit according to Claim 11 or 12, d) a plunger configured to be inserted into the blood collection tube to force blood out of the capillary tube.

15. The kit according to Claim 11 or 12, further comprising e) a blood collection puncture device.

16. A method for transporting or storing microvolume blood, the method comprising:
providing the solution according to any one of Claims 1 to 9;
collecting microvolume blood; and
preparing a diluent solution by mixing the microvolume blood with the solution.

17. The method according to Claim 16, wherein the microvolume blood that is mixed with the solution is whole blood.

18. The method according to Claim 17, further comprising transporting the prepared diluent solution from a location at which the microvolume blood was collected to a different location.

19. A method comprising:
providing a blood collection tube comprising a capillary tube and a cylinder fluidly connected to the capillary tube;
providing a tube holding a diluent solution;
collecting fingertip blood into the blood collection tube comprising the capillary tube;
introducing the blood collection tube, which holds the fingertip blood, into the tube in a manner that allows an end of the capillary tube to come into contact with the diluent solution;
waiting for the fingertip blood present in the blood collection tube to drop into the diluent solution;
inserting a plunger into the cylinder to force out a liquid present in the capillary tube;
removing the plunger and the blood collection tube from the tube; and
closing the tube to seal a mixed solution of the diluent solution and the fingertip blood in the tube.

## Amended claims

### Amended claims under Art. 19.1 PCT

1. (Amended) A kit for sending microvolume blood by postal mail or for storing microvolume blood, the kit comprising:
a blood collection tube comprising a capillary tube and a cylinder fluidly connected to the capillary tube, the capillary tube having an end opening that is brought into contact with capillary blood to collect a predetermined volume of microvolume blood; and
a tube containing a diluent solution that comprises a buffering agent and a salt as active ingredients and dilutes the microvolume blood at a dilution ratio of greater than 8, wherein
the blood collection tube is configured to be inserted with the capillary tube being positioned downward and is also configured to allow the end opening of the blood collection tube to reach a surface of the diluent solution to enable blood held in the capillary tube to exit into the diluent solution through the end opening.

2. (Deleted)

3. (Amended) The kit according to Claim 1, wherein the microvolume blood is whole blood.

4. (Amended) The kit according to Claim 1, wherein the predetermined volume of the microvolume blood that is collected is 0.5 to 20 µL.

5. (Amended) The kit according to Claim 1, wherein the dilution ratio is substantially 10 or greater.

6. (Amended) The kit according to Claim 1, wherein the buffering agent is selected from the group consisting of HEPES, sodium acetate, and PBS.

7. (Amended) The kit according to Claim 1, wherein the salt is selected from the group consisting of NaCl, KCl, MgCl, and CaCl.

8. (Amended) The kit according to Claim 1, wherein the active ingredients are adjusted such that the diluent solution forms an isotonic solution with respect to a blood cell component.

9. (Amended) The kit according to Claim 1, wherein the kit is used to measure a GA value, the GA value being measured after the collected microvolume blood is transported or is stored for a storage period.

10. (Deleted)

11. (Deleted)

12. (Deleted)

13. (Amended) The kit according to Claim 1, wherein an amount of the diluent solution is 90 µL, and the volume of blood collectable by the blood collection tube is 10 µL.

14. (Amended) The kit according to Claim 1, further comprising d) a plunger configured to be inserted into the blood collection tube to force blood out of the capillary tube.

15. (Amended) The kit according to Claim 1 or 14, further comprising e) a blood collection puncture device.

16. (Amended) A method for transporting or storing microvolume blood, the method comprising:
providing the kit according to any one of Claims 1 to 15;
collecting microvolume blood; and
preparing a diluent solution by mixing the microvolume blood with the diluent solution.

17. The method according to Claim 16, wherein the microvolume blood that is mixed with the solution is whole blood.

18. The method according to Claim 17, further comprising transporting the prepared diluent solution from a location at which the microvolume blood was collected to a different location.

19. (Amended) A method for sending microvolume blood by postal mail, the method comprising:
providing the kit according to any one of Claims 1 to 15;
collecting microvolume blood into the capillary tube of the blood collection tube;
introducing the blood collection tube, which holds the microvolume blood, into the tube in a manner that allows an end opening of the capillary tube to come into contact with a surface of the diluent solution;
waiting for the microvolume blood present in the blood collection tube to exit into the diluent solution;
inserting a plunger into the cylinder to force out a liquid present in the capillary tube;
removing the plunger and the blood collection tube from the tube; and
closing the tube to seal a mixed solution of the diluent solution and the microvolume blood in the tube.

Statement under Art. 19.1 PCT
1. Independent Claim 1:
It is explicitly stated, based on original Claim 2, that the blood to be collected is capillary blood, and original Claim 2 has been canceled.
The "solution" has been amended to the "kit" based on original Claim 10. The "tube" is explicitly recited based on original Claim 11. The "blood collection tube" is explicitly recited based on original Claim 12. Original Claims 10 to 12 have been canceled.
The statements "a blood collection tube comprising a capillary tube and a cylinder fluidly connected to the capillary tube, the capillary tube having an end opening that is brought into contact with capillary blood to collect a predetermined volume of microvolume blood" and "the blood collection tube is configured to be inserted with the capillary tube being positioned downward and is also configured to allow the end opening of the blood collection tube to reach a surface of the diluent solution to enable blood held in the capillary tube to exit into the diluent solution through the end opening" are based on paragraphs [0043] and [0044] of the specification of the present application.

2. The subject matters of Claims 3 to 9 have been amended to the "kit", as with Claim 1.

3. Claims 4, 9, 13, and 16 have been amended in accordance with the amendment to Claim 1, which is cited in the foregoing claims.

4. Independent Claim 19 has been amended in a manner similar to that of independent Claim 1.
 Applicants respectfully submit that the amendments described above do not constitute the addition of new matter and are legitimate.
